# NEW EUROPEAN PATENT SPECIFICATION

(11) **EP 2 295 529 B2**
(45) Date of publication and mention of the opposition decision: **18.05.2022**
(45) Mention of the grant of the patent: 27.05.2015
(21) Application number: 10185785.2
(22) Date of filing: 08.07.2003
(51) Int. Cl.: C11B 3/12, A23D 9/00, A23K 1/16, A23K 1/18, C11B 3/14, C11C 3/10, C11B 3/00

(54) **Use of a volatile environmental pollutants-decreasing working fluid for decreasing the amount of pollutants in a fat for alimentary or cosmetic use**
Verfahren zur Verminderung von Umweltschadstoffen in einem Öl oder einem Fett und Fischfutterprodukt
Procédé de diminution des polluants environnementaux dans une huile ou une graisse et produit d'alimentation pour poissons

(30) Priority: 11.07.2002 SE 0202188
(43) Date of publication of application: 16.03.2011
(62) Divisional of application: 09180207.4
(73) Proprietor: BASF AS, 1327 Lysaker (NO)
(72) Inventor: Breivik, Harald, 8140 Inndyr (NO); Thorstad, Olav, 3942, Porsgrunn (NO)
(74) Representative: Weickmann & Weickmann PartmbB

(56) References cited:
- WO-A1-95/24459
- DE-A1- 3 839 017
- JULSHAMN K ET AL: "Removal of DDT and its metabolites from fish oils by molecular distillation", FISKERIDIREKTORATETS SKRIFTER. SERIE. TEKNOLOGISKE UNDERSOKELSER- REPORT ON TECHNOLOGICAL RESEARCH CONCERNING NORWEGIAN FISHINDUSTRY, DIRECTORATE OF FISHERIES, BERGEN, NO, vol. 5, no. 15, 1 January 1978 (1978-01-01), pages 3-11, XP002971951, ISSN: 0078-186X
- BIMBO A P: "Fish Oils in Nutrition", FISH OILS: THEIR CHEMISTRY, TECHNOLOGY, STABILITY, NUTRITIONALPROPERTIES AND USES, AVI PUBLISHERS, WESTPORT, CN, US, 1 January 1990 (1990-01-01), pages 181-225, XP003008145,
- BIMBO ANTHONY P: "GUIDELINES FOR CHARACTERIZING FOOD-GRADE FISH OIL", INFORM, AOCS, AMERICAN OIL CHEMISTS SOCIETY, CHAMPAIGN, IL, US, vol. 9, no. 5, 1 January 1998 (1998-01-01) , pages 473-483, XP008078334, ISSN: 0897-8026
- KANEMATSU H ET AL: "STUDIES ON THE BEHAVIOR OF TRACE COMPONENTS IN OILS AND FATS DURING PROCESSING FOR EDIBLE USE. I. REMOVAL OF ORGANOCHLORINE PESTICIDES AND POLYCHLORINATED BIPHENYLS FROM OILS AND FATS BY PROCESSING FOR EDIBLE USE", YUKAGAKU, NIHON YUKAGAKU KYOKAI, TOKYO, JP, vol. 25, no. 1, 1 January 1976 (1976-01-01), pages 38-41, XP001183221, ISSN: 0513-398X & KANEMATSU H ET AL: "Behaviour of trace components in oils and fats during processing for edible use. I. Removal of organochlorine pesticides and polychlorinated biphenyls (PCB) from oils and fats", FSTA, 1 January 1976 (1976-01-01), XP002085013,
- Cynthia A De Wit: "An overview of brominated flame retardants in the environment", Chemosphere, vol. 46, no. 5, 1 February 2002 (2002-02-01), pages 583-624, XP055103677, ISSN: 0045-6535, DOI: 10.1016/S0045-6535(01)00225-9

## Description

### TECHNICAL FIELD OF THE INVENTION

This invention relates to a process for decreasing the amount of environmental pollutants in a mixture comprising a fat or an oil, being edible or for use in cosmetics. The present invention also relates to a volatile environmental pollutants decreasing working fluid. In addition, the present invention relates to a health supplement, a pharmaceutical, a cosmetic product and an animal feed product prepared according to the process mentioned above.

### BACKGROUND OF THE INVENTION

DDT (2,2 bis-(p-chlorophenyl)-1,1,1-trichloroethane) and its degradation products are today found almost everywhere in the global environment. Numerous studies also report on the accumulation of often relatively high concentrations of environmental pollutants like PCB, dioxins and brominated flame retardants, and pesticides like toxaphenes and DDT and its metabolites in the deposit of e.g. marine organisms. The hazard of these compounds for both humans and animals have caused a growing concern about the content of toxic substances in food and food stuff. Consumption of dioxins above safe levels over a lifetime may result in an increased risk of cancer.

Food products that have no or reduced amounts of pollutants are gaining popularity as well as an increasing share of the market. Consequently, removal or reduction of pollutants in food products have the potential to substantially increase marketability and value.

The commercially important polyunsaturated fatty acids in marine oils, such as fish oil, are preferably EPA (eicosapentaenoic acid, C20:5n-3) and DHA (docosahexaenoic acid, C22:6n-3). The full nomenclature of these acids according to the IUPAC system is: EPA all-cis-5,8,11,14,17-eicosapentaenoic acid, DHA all-cis-4,7,10,13,16,19-docosahexaenoic acid. For many purposes it is necessary that the marine oils should be refined in order to increase the content of EPA and/or DHA to suitable levels, or to reduce the concentrations of, or even eliminate, certain other substances which occur naturally in the raw oil.

The fatty acids EPA and DHA are also proving increasingly valuable in the pharmaceutical and food supplement industries in particular. It is also very important for fish oils and other temperature sensitive oils (e.g. oils that contain long chain polyunsaturated fatty acids) to keep the temperature in some of the processes as low as possible.

The demand for marine oils of high quality is increasing. This issue forces the fish oil industry to consider use of alternative refining techniques for fish oils with inferior quality, i.e. oils with high amounts of free fatty acids that make the oils less useful for nutritional purposes and make traditional alkaline refining more complicated and costly. If environmental pollutants can be successfully removed from such fish oils they are appropriate for use in the animal feed industry, e.g. in animal feed products.

From the literature it is known that molecular distillation, or short path distillation as the technique alternatively may be named, can be used to remove the pesticides DDT and its metabolites from fish oil (K. Julshamn, L. Karlsen and O.R. Braekkan, Removal of DDT and its metabolites from fish oils by molecular distillation, Fiskeridirektoratets skrifter; Serie teknologiske undersøkelser, Vol. 5 No. 15 (1973)). A practical upper limit was 65% removal together with a loss of about 25% of vitamin A. In many industrial fish oil refining processes a removal of DDT up to 65% is not satisfactory.

Anthony P. Bimbo: Guidelines for characterization of food-grade fish oil. INFORM 9(5), 473-483 (1998), reported that vacuum stripping or thin-film distillation can be used to remove chlorinated hydrocarbons and free fatty acids from fats or oils. A disadvantage by using vacuum stripping to refine oils is that sufficient results only can be achieved then the vacuum stripping process is carried out at a high temperature. Further, the high temperature gives rise to undesirable side reactions.

Jiri Cmolik og Jan Pokorny: Physical refining of edible oils, Eur. J. Lipid Sci. Technol. 102(7), 472-486 (2000) describes physical refining of edible oils and the use of molecular distillation for removal of undesirable substances in crude oils, preferably crude vegetable oils, respectively the use of steam stripping in order to remove free fatty acids from an oil composition. Physical refining is used to refine oils of good quality, i.e. oils with small amounts of free fatty acids. However, physical refining is more complicated and costly for oils with inferior quality.

In WO 9524459 a process for treating an oil composition containing saturated and unsaturated fatty acids in the form of triglycerides, in order to obtain a refined product with higher concentrations of the polyunsaturated fatty acids, is presented. This process also is intended to be used for removal of some environmental pollutants from an oil composition, wherein the process comprises the steps of; subjecting the oil composition to a transesterification reaction and thereafter subjecting the product obtained in the first step to one or more molecular distillations. This technique has the severe limitation that it can only be used for fish oils that have been partially transesterified using a lipase catalyst that discriminates against omega-3 fatty acids. Obviously, this technique can not be used for commercial fish oils.

In EP0632267 A1 a method of measuring the content of polycyclic aromatic hydrocarbons(PAH) remaining in lanolin is presented. The European patent document also describes a method of removing PAH remaining in wool grease or lanolin by a vacuum distillation of the grease or lanolin under specified conditions either directly or after having been treated with a borate and, if necessary, obtaining various lanolin derivatives from the treated wool grease or lanolin. However, the technique described in said patent document requires very high temperatures (230°C) in order to achieve 90% reduction in PAH content.

Another interesting observation is that the removal of environmental pollutants from fats or oils is not a trivial matter. Several different techniques, some of which are mentioned above, to accomplish this task have been developed, but none of them is sufficiently effective and gentle to the fat or oil. In addition, it is nowadays a problem for e.g. the marine oil industry that the amounts of pollutants in e.g. fish oil become increased.

### SUMMARY OF THE INVENTION

The scope of the invention is defined by the appended claims.
One object of the present invention is to offer an effective process for decreasing the amount of environmental pollutants in a fat or an oil, being edible or for use in cosmetics.

According to a first aspect, this and other objects are achieved with a process for decreasing the amount of environmental pollutants in a mixture comprising a fat or an oil, being edible or for use in cosmetics, the fat or oil containing the environmental pollutants, which process comprises the steps of adding a volatile working fluid to the mixture, where the volatile working fluid consisting of a fatty acid ethyl ester, a fatty acid methyl ester or a fatty acid amide obtained from at least one of vegetable, microbial and animal origin, or any combination thereof, and subjecting the mixture with the added volatile working fluid to at least one stripping processing step, in which an amount of environmental pollutants present in the fat or oil, being edible or for use in cosmetics, is separated from the mixture together with the volatile working fluid. Herein, "an amount" is interpreted to include decreasing of an amount up to 95-99% of some environmental pollutants, i.e. a substantial removal of specific pollutants and/or toxic components from a fat or oil composition.

The use of a volatile working fluid in a stripping process for decreasing an amount of environmental pollutants in a mixture comprising a fat or an oil, being edible or for use in cosmetics, containing the environmental pollutants, and/or toxic components, has a number of advantages.

An advantage of using a volatile working fluid in a process comprising at least one stripping processing step is that an amount of environmental pollutants in the mixture can more easily be stripped off together with the volatile working fluid, i.e. the environmental pollutants present in the fat or oil mixture is separated from the mixture together with the working fluid. Preferably this is possible as long as the volatile working fluid is essentially equally or less volatile than the environmental pollutants that is to be removed from the fat or oil mixture. The stripped pollutants (components) and most of the volatile working fluid will be found in the distillate.

In addition, the use of a volatile working fluid as defined above in at least one stripping process step results in that use of the inventive process decreases the amount of dioxins in a fish oil with more than 95%. By using the inventive process it is also possible to decrease the amount of chlorinated organic pesticides (or pollutants) in a mixture comprising a fat or an oil, being edible or for use in cosmetics, which pollutants are even less volatile than DDT, for instance dioxines, toxaphenes and/or PCB. Separation of such heavy and undesirable components from the fat or oil mixture according to the invention, using mild conditions that do not decompose even very unsaturated oils, is surprising. Further, according to the present stripping process it is possible to decrease an effective amount of PAH at much lower temperatures compared to the techniques known from the prior art.

Another advantage of adding a volatile working fluid to an oil or fat mixture prior to a stripping process is that removal of free fatty acids is facilitated, which will result in a higher quality of the oil product.

In addition, the volatile working fluid according to the invention allows environmental pollutants or other toxic components to be stripped off by e.g. molecular distillation even from oils of lower quality, i.e. oil for feed purposes. Further, the process according to the invention can also be used for decreasing the amount of toxic compounds in a ricinus oil, preferably trace of ricinine (1,2-dihydro-4-methoxy-1-methyl-2-oxo-3-pyridinecarbonitrile). By using the process according to the invention the amount of ricinine may be decreased with at least 80-90 %.

In another preferred embodiment the volatile working fluid comprises at least one fatty acid ester composed of C10-C22 fatty acids and C1-C2 alcohols, or a combination of two or more fatty acid ester each composed of C10-C22 fatty acids and C1-C2 alcohols. Preferably, the volatile working fluid is at least one of amides composed of C10-C22 fatty acids and C1-C4 amines. Most preferably, the volatile working fluid is a mixture of ethyl or methyl esters of marine fatty acids from marine oils, e.g. fish body oil and/or fish liver oil.

The fatty acid esters and fatty acid amides are obtained from at least one of vegetable, microbial and animal fat or oil. The fatty acid esters mentioned above can e.g. be a by- product from distillation of an ethyl ester mixture prepared by ethylation of preferably a fish oil. In the process industry trade with intermediates is increasing and opens up for an extra financial income.

In another preferred embodiment the volatile working fluid is obtained from at least one of animal fat or oil, wherein the animal fat or oil preferably is a marine oil e.g. a fish oil or an oil from other marine organism e.g. sea mammals.

Further, in another preferred embodiment of the invention the fat or oil, being edible or for use in cosmetics, is obtained from at least one of vegetable, microbial and animal fat or oil, or any combination thereof. Preferably, the fat or oil, being edible or for use in cosmetics, is a marine oil. Marine oils that have no or reduced amounts of environmental pollutants are gaining popularity as well as an increasing share of the market. Consequently, removal or reduction of pollutants in e.g. fish oils of high quality as well as fish oils with inferior quality have the potential to substantially increase marketability and value. Therefore, in a more preferred embodiment of the invention the marine oil is obtained from fish or sea mammals, containing at least saturated and unsaturated fatty acids in the form of triglycerides. It is important to note that the invention is not limited to procedures were the working fluid is prepared from the same origin as the oil that is being purified.

Additionally, the fat or oil, being edible or for use in cosmetics, may also be a ricinus oil for use in cosmetics or medicinal applications. It is also of commercial interest to decrease the amount of pollutants or toxic components in oil mixtures or blends comprising at least one microbial oil that e.g. will be used in food products or as food supplement (e.g. infant formula) preferable suitable for humans.

In another preferred embodiment of the invention the fat or oil, being edible or for use in cosmetics, is a tocopherol concentrate prepared from a condensate from at least one deodorization process of at least one vegetable oil, wherein the tocopherol concentrate containing at least one of PAH and volatile pollutants, or any combination thereof. Commercially available tocopherol concentrate contains about 65-90% tocopherol and it will be apparent for one skilled in the art that the stripping process according to the invention may be used to separate an amount of environmental pollutants from a tocopherol concentrate.

In a preferred embodiment of the invention, the ratio of (volatile working fluid):(fat or oil, being edible or for use in cosmetics) is about 1:100 to 15:100. In a more preferred embodiment the ratio of (volatile working fluid):(fat or oil, being edible or for use in cosmetics) is about 3:100 to 8:100.

The stripping process step is carried out at temperatures in the interval of 150-270 °C.

In a most preferred embodiment, the stripping processing step is carried out at temperatures in the interval of 150-200 °C. By adding a volatile working fluid to the fat or oil mixture at this temperatures the invention surprisingly shows that even termolabile polyunsaturated oils can be treated with good effect, without causing degradation of the quality of the oil.

In another preferred embodiment, the stripping processing step is carried out at a pressure below 1 mbar.

In another preferred embodiment, the stripping processing step is at least one of a molecular distillation or a short-path distillation, or any combination thereof. By using a stripping process, e.g. a distillation method, for decreasing the amount of environmental pollutants in a fat or oil mixture comprising a volatile working fluid it is possible to carry out the stripping processes at lower temperatures, which spare the oil and is at the same time favourable to the end oil product.

In a preferred embodiment of the invention, the volatile working fluid is stripped off together with the environmental pollutants by at least one short-path distillation or molecular distillation step. This is possible as long as the volatile working fluid is essentially equally or less volatile than the environmental pollutants that are to be separated from the fat or oil mixture.

In another preferred embodiment of the invention, the process allows the environmental pollutants to flash off most effective at process conditions of low temperatures and preferable high mixture flow rates. Further, this embodiment offers similar advantages as described above by using the volatile working fluid.

In a preferred embodiment according to the invention the stripping process is carried out by a molecular distillation in the following intervals; mixture flow rates in the interval of 10-300 kg/h·m², temperatures in the interval of 120-270 °C and a pressure below 1 mbar.

In a most preferred embodiment of the invention the molecular distillation is carried out at temperatures in the interval of 150-200°C and at a pressure below 0,05 mbar.

In another preferred embodiment of the present invention, for use in decreasing an amount of environmental pollutants and/or toxic components, such as dioxins and/or PCB, present in a fat or oil, being edible or for use in cosmetics, the working fluid is as defined above with essentially equally or less volatility compared to the environmental pollutants that are to be separated from the fat or oil mixture, or any combination thereof.

Preferably, the volatile environmental pollutants decreasing working fluid is generated as a fractionation product. Additionally, the volatile environmental pollutants decreasing working fluid is a by-product, such as a distillation fraction, from a regular process for production of ethyl and/or methyl ester concentrates. This by-product according to the invention can be used in a new process for decreasing the amount of environmental pollutants in a fat or an oil. More preferably, the volatile environmental pollutants decreasing working fluid, for use in decreasing an amount of environmental and/or toxic components, present in a fat or oil, can be a by-product (a distillate fraction) from a regular process for production of ethyl ester concentrates, wherein a mixture comprising an edible or a non-edible fat or oil, preferably a fish oil, is subjected to an ethylating process and preferably a two-step molecular distillation. In the two-step molecular distillation process a mixture consisting of many fatty acids on ethyl ester form is separated from each other in; a volatile (light fraction), a heavy (residuum fraction) and a product fraction. The volatile fraction from the first distillation is distilled once more and the volatile fraction from the second distillation process is then at least composed of the volatile working fluid, preferably a fatty acid ethyl ester fraction. This fraction consists of at least one of C14 and C16 fatty acids and at least one of the C18 fatty acids from the fat or oil, and is therefore also compatible with the edible or non-edible oil. The fraction can be redistilled one or more times if that is deemed to be suitable. This prepared working fluid can then be used as a working fluid in a new process for decreasing the amount of environmental pollutants in a fat or an oil, wherein the edible or non-edible fats or oils and the oil or fat, being edible or for use in cosmetics, are of the same or different types.

In another embodiment of the invention a volatile working fluid may be produced by subjecting fats or oils from an available source, for instance fats or oils obtained from at least one of animal, microbial or vegetable origin, to an inter-esterification process, in which process the triglycerides in the fats or oils are converted into esters of aliphatic alcohols. Additionally, a bio-diesel can be used as a volatile working fluid. In the case when the volatile working fluid is a biodiesel, it can be produced by a process, which is in common use for production of engine fuels (biodiesel), and therefore also known by a man skilled in the art, which process comprises mixing the fat or oil with a suitable amount of aliphatic alcohol, adding a suitable catalyst and heating the mixture for a period of time. Similar esters of aliphatic alcohols may also be produced by a high-temperature catalytic direct esterification process reacting a free fatty acid mixture with the appropriate aliphatic alcohol. The fatty acid ester mixture produced in this manner may be used as a volatile working fluid as it is, but normally the conversion to esters of aliphatic alcohols is not complete, the conversion process preferably leaving some un-reacted non-volatile glycerides in the mixture. Additionally, some fats or oils may also contain certain amounts of non-volatile, non-glyceride components (e.g. polymers). Such non-volatile components will preferably be transferred to, and mixed with the final product, which product is low in environmental pollutants, when the fatty acid ester mixture is used as working fluid. A working fluid produced in this manner should therefore be subjected to a distillation, preferably a molecular and/or short path distillation, in at least one step, which distillation process generates a distillate more suitable to be used as a new volatile working fluid.

In another preferred embodiment of the invention the volatile working fluid comprises at least one of an ester and/or an amide composed of shorter fatty acids and longer alcohols or amines, or any combination thereof.

The volatile environmental pollutants decreasing working fluid, for use in decreasing an amount of environmental pollutants present in a fat or oil, being edible or for use in cosmetics, is a fatty acid ethyl ester or fatty acid methyl ester or a fatty acid amide obtained from at least one of vegetable, microbial and animal origin, or any combination thereof. Preferably, said animal origin is fish or sea mammals, i.e. that the volatile fat or oil environmental pollutants decreasing working fluid is obtained from marine oils, e.g. from fish or from sea mammals. Further, in a preferred embodiment of the volatile environmental pollutants decreasing working fluid, said fat or oil is edible for humans and/or animals or for use in cosmetics.

In another embodiment of the invention, a volatile environmental decreasing working fluid according to the present invention, is used in a process for decreasing an amount of environmental pollutants, in a mixture comprising a fat or oil, being edible or for use in cosmetics, preferably a marine oil, containing the unwanted components, in which process the volatile working fluid is added to the mixture and then the mixture is subjected to at least one stripping processing step, which is a molecular distillation or a short-path distillation, or any combination thereof, and in which process an amount of toxic components present in the fat or oil, being edible or for use in cosmetics, is separated from the mixture together with the volatile working fluid.

In another preferred embodiment a health supplement, a pharmaceutical and/or an animal feed product containing at least fat or oil (end) products, e.g. oil ingredient of fish feed, with a decreased amount of environmental pollutants or toxic components, prepared according to at least one of the previously mentioned processes is disclosed. For the pharmaceutical and food supplement industries, marine oils have to be processed in order to increase the content of EPA and/or DHA to suitable levels and the removal or reduction of different kinds of pollutants have the potential to substantially increase marketability and value. Therefore, the present invention also discloses a health supplement and a pharmaceutical respectively, containing at least a marine oil, such as fish oil, which marine oil is prepared according to the previously mentioned process, in order to decrease the amount of environmental pollutants in the marine oil.

In another embodiment the pharmaceutical and/or health supplement is preferably intended for treating cardiovascular diseases (CVD) and inflammatory diseases, but they also have positive effects on other CVD risk factors such as the plasma lipid profile, hypertension and vascular inflammation. In more preferred embodiment of the invention the pharmaceutical and/or health supplement comprises at least one of EPA/DHA ethyl esters and is intended for a range of potential therapeutic applications including; treatment of hypertriglyceridaemia, secondary prevention of myocardial infarction, prevention of atherosclerosis, treatment of hypertension and/or kidney disease and to improve children's learning ability.

Further disclosed is a marine oil product, prepared according to at least one of the previously mentioned processes. Preferably, the marine oil product is based on fish oil or a fish oil composition.

In another preferred embodiment, an animal feed product, containing at least a marine oil, which marine oil is prepared according to one of the processes presented before, in order to decrease the amount of environmental pollutants and/or the amount of free fatty acids in the marine oil. Preferably the animal feed product is a fish feed product.

For companies producing tocopherol preparations it is of commercial interest to refine their tocopherol concentrate from environmental pollutants, especially PAH's (polycyclic aromatic hydrocarbons). Tocopherol is produced from condensate from deodorization of soy and/or palm oil. Vegetable oils are deodorized preferably in the same way as fish oils, but at higher temperatures in order to distil off the tocopherols. Therefore, the condensate from the mentioned process also contains, except components causing bad taste and odour of the oil, high amounts of tocopherol. Further, this condensate is raw material in all tocopherol preparations that are so-called natural preparations.

In another preferred embodiment a tocopherol concentrate product, based on a tocopherol concentrate prepared from a condensate from a deodorization process of at least one vegetable fat or oil, such as palm oil or soy oil, which concentrate contains at least one of PAH and volatile pollutants, is prepared according to the stripping process of the present invention, in order to decrease the amount of environmental pollutants in the tocopherol concentrate. The process for decreasing the amount of environmental pollutants and/or PAH's in a tocopherol concentrate, comprises the steps of adding a volatile working fluid to a tocopherol concentrate and subjecting the concentrate, with the added volatile working fluid, to a stripping processing step, in which preferably PAH's are separated from the concentrate with the volatile working fluid. The volatile working fluid may be at least one of the working fluids mentioned before, or any combinations thereof, and said stripping process step is carried out at process conditions mentioned before. In a embodiment of the invention said stripping process step is carried out at a temperature in the interval of 120-180 °C and at a pressure below 1 mbar. The ratio of (volatile working fluid):(toco-pherol concentrate) is preferably about 4:100 to 8:100.

For the cosmetic industry it is of commercial interest to refine ricinus oil from trace of toxic components. It is valuable for this industry to market cosmetic products, such as lipstick, that are essentially free from toxic components, such as ricinine. Ricinus oil is a vegetable oil produced from the seeds of *Ricinus communis* L., *Euphorbiaceae.* The oil is a triglyceride of fatty acids, with ricinoleic acid (d-12-hydroxyoleic acid) as the major fatty acid (approximately 87%). Due to the hydroxyl group of ricinoleic acid, ricinus oil can not be refined in traditional ways, i.e. by alkali refining. Thus, traces of toxic components may not be easily removed. Ricinine is a toxic nitrilpyridinone that might be present in the oil in trace amounts. Herein, the invention also disclose a cosmetic product, based on ricinus oil, which ricinus oil is prepared according to at least one of the processes mentioned before, in order to decrease the amount of toxic components in the ricinus oil.

In another preferred embodiment of the invention the fat or oil is a ricinus oil, for use in cosmetics or medicinal applications, and the pollutants that are to be separated according to the process of the invention are toxic compounds, such as nitrilpyridinones.

In a preferred embodiment, the process for decreasing the amount of toxic compounds in a ricinus oil, preferably trace of ricinine, comprises the steps of adding a volatile working fluid to a ricinus oil mixture and subjecting the mixture, with the added volatile working fluid, to a stripping processing step, in which preferably traces of ricinine (from the ricinus oil) is separated from the mixture with the volatile working fluid. The volatile working fluid may be at least one of the working fluids mentioned before, or any combinations thereof.

In a preferred embodiment, the ricinus oil mixture was distilled at a temperature about 170 °C, a pressure around 0,001 mbar respectively a mixture flow rate about 150 kg/h·m². Up to 95% of the amount of ricinine present in the start oil may be removed with the stripping process according to the invention.

### DEFINITIONS

As used herein the term environmental pollutants preferably means toxic components and/or pesticides like polychlorinated biphenyls (PCB), DDT and its metabolites, organic compounds found in the sea environment and identified as potentially harmful and/or toxic; Polychlorinated triphenyls (PCTs), dibenzo-dioxins (PCDDs), and dibenzo-furans (PCDFs), Chlorophenols and hexachlorocyclohexanes (HCHs), toxaphenes, dioxins, brominated flame retardants, polyaromatic hydrocarbons (PAH), organic tin-compounds (e.g. tributyltin, triphenyltin) and organic mercury-compounds (e.g. Methyl-Mercury).

As used herein the term oil and fat means fatty acids in at least one of the triglyceride_and phospholipid forms. Generally, if the start material in the stripping process is a marine oil, the oil may be any of raw or partially treated oil from fish or other marine sources and which contains fatty acids, including polyunsaturated fatty acids, in the form of triglycerides. Typically, each triglyceride molecule in such a marine oil will contain, more or less randomly, different fatty acid ester moieties, be the saturated, monounsaturated or polyunsaturated, or long chain or short chain. Further, examples of vegetable oils or fats are corn oil, palm oil, rapeseed oil, soybean oil, sunflower oil and olive oil. Further, the fat or oil may be pre-processed in one or several steps before constituting the start material in the stripping process as described above. An example of such a pre-processing step is a deodorization process. It shall also be noted that the fat or oil may be edible in one or several such pre-processing steps and/or in the processing steps according to the invention.

As used herein the term edible means edible for humans and/or animals. Additionally, as used herein the term "for use in cosmetics" means an oil or a fat that can be used in products that contributes to enhance humans appearance and/or health, e.g. cosmetic and/or beauty care products.

As used herein the term working fluid is interpreted as defined above.

As used herein the term essentially equally or less volatile is interpreted to include that the volatile working fluids having a suitable volatility in relation to the volatility of the environmental pollutants that is to be stripped off from a fat or oil mixture. Further, commonly this is the case when the volatility of the working fluid is the same or lower than the volatility of the environmental pollutants. However, the term essentially equally or less volatile is also intended to include the case when the volatile working fluid is somewhat more volatile than the environmental pollutant.

As used herein the term "oils with a low quality" preferably means that the oil contains high amounts of free fatty acids, that makes them less useful for nutritional purposes and that traditional alkaline refining in such oils is complicated and costly.

As used herein bio-diesel means a commercial product (or products under development) used as an environment friendly alternative to fuel for cars comprising e.g. methyl esters from preferable vegetable or animal oils.

As used herein the term marine oils includes oil from fish, shellfish (crustaceans) and sea mammals. Non limiting examples of fish oils are e.g. Menhaden oil, Cod Liver oil, Herring oil, Capelin oil, Sardine oil, Anchovy oil and Salmon oil. The fish oils mentioned above may be recovered from fish organs, e.g. cod liver oil, as well as from the meat of the fish or from the whole fish.

As used herein the term health supplement is interpreted to include food and food supplement to animals and/or humans, fortification of food, dietary supplement, functional (and medical) food and nutrient supplement.

As used herein the term "treating" means both treatment having a curving or alleviating purpose and treatment having a preventive purpose. The treatment can be made either acutely or chronically. Herein the term animal feed product means food or food supplement specially to animals e.g. fish, fowls, pigs and furred (fur-bearing) animal.

As used herein the term fish feed product also includes a fish larvae feed product.

As used herein the term microbial oils also includes "single cell oils" and blends, or mixtures, containing unmodified microbial oils. Microbial oils and single cell oils are those oils naturally produced by microorganisms during their lifespan.

Further, a fat or an oil, being edible or for use in cosmetics, according to the invention can also be a blend of e.g. microbial oils, fish oils, vegetable oils, or any combination thereof.

As used herein the term free fatty acids means fatty acids in free acid form.

As used herein the term "together with", means that the volatile working fluid will be stripped off together with, combined with, or adhering the pollutants, namely that the pollutants will accompany the working fluid.

As used herein the term acid value of a fat or an oil means the amount of free acids presented in a fat or an oil equal to the number of milligrams of potassium hydroxide needed to neutralize one gram of the oil, i.e. that the term serves as an index of the efficiency of refining. This means that a high acid value is characteristic for low quality oil or fat products.

### BRIEF DESCRIPTION OF THE DRAWINGS

The advantages and details of the present invention will become apparent from the following description when taken in conjugation with the accompanying drawings, in which;

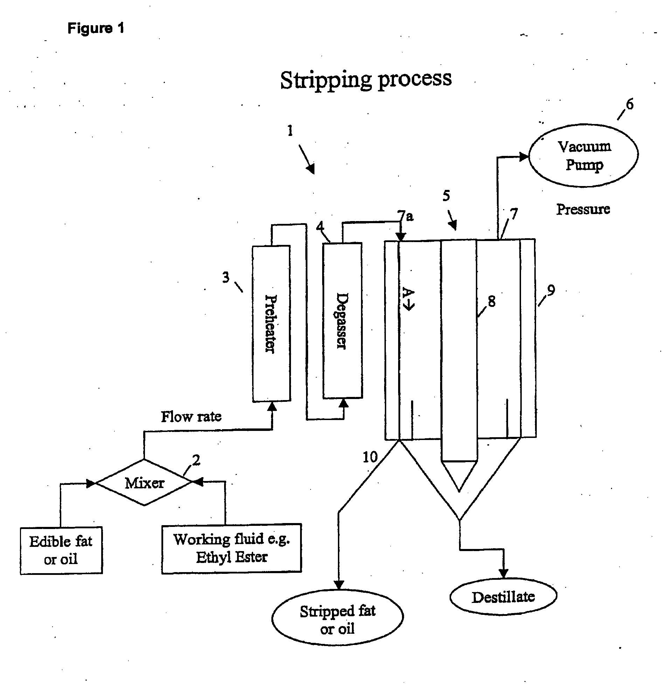

FIG 1 is a schematic flow chart of one embodiment illustrating a method for decreasing the amount of environmental pollutants in a fat or an oil, being edible or for use in cosmetics, by adding a volatile working fluid prior to a molecular distillation.

### DETAILED DESCRIPTION OF PREFERRED EMBODIMENTS

A number of preferred embodiments of the process for decreasing the amount of environmental pollutants in a mixture comprising a volatile working fluid and a fat or an oil, being edible or for use in cosmetics, containing environmental pollutants will be disclosed below.

A first embodiment of a process for decreasing the amount of environmental pollutants in a fat or an oil, being edible or for use in cosmetics, by adding a volatile working fluid prior to a molecular distillation is presented in figure 1. The starting fat or oil, being edible or for use in cosmetics, in the first embodiment of the invention is a fish oil whether freshly refined, reverted or mixtures thereof characterized by a level of environmental pollutants. The exact amount of environmental pollutants varies depending upon such factors as fish species, seasonality, geographical catch location and the like.

As used herein the term molecular distillation is a distillation process performed at high vacuum and preferably low temperature. Herein, the condensation and evaporation surfaces are within a short distance from each other, so as to cause the least damage to the oil composition. This technique is also called short-path distillation, and commercial equipment is readily available.

The molecular distillation plant (1) illustrated in figure 1, comprises a mixer (2), a pre-heater (3), a degasser (4), a distillation unit (5) and a vacuum pump (6). In accordance to this embodiment, a volatile working fluid comprising an ethyl ester fraction (6% relative to the oil) is added to a fish oil mixture and blended in a mixer (2). The oil mixture is then optionally passed through a means (3) for controlling the oil feed rate (herein about 400 kg/h), such as an ordinary throttling valve. The fish oil mixture is then preheated with a heating means (3) such as a plate heat exchanger to provide a preheated fish oil mixture. The mixture is then passed through a degassing step (4) and admitted into the molecular path distance evaporator (5), a tube (7) including the condensation (8) and evaporation (9) surface. The stripping process is carried out at a pressure between 0,1 and 0,001 mbar and at a temperature of about 200 °C. The fish oil mixture to be concentrated is picked up as it enters the tube (7a) by rotating blades. The blades extend nearly to the bottom of the tube and mounted so that there is a clearance of about 1,3 mm between their tips and the inner surface of the tube. In addition, the blades are driven by an external motor. The fish oil mixture is thrown against the tube wall and is immediately spread into a thin film and is forced quickly down the evaporation surface. The film flows down by gravity and becomes concentrated as it falls. Heated walls and high vacuum strips off the volatile working fluid together with the environmental pollutants, i.e. the more volatile components (distillate) is derived to the closely positioned internal condenser (8), the less volatile components (residue) continues down the cylinder. The resulting fraction, the stripped fish oil mixture containing at least the fatty acids EPA and DHA is separated and exit through an individual discharge outlet (10).

In a third embodiment of the invention the process is carried out by a short path distillation, which includes the use of a short path evaporator that integrates the features and advantages of thin film or wiped film evaporators but adds internal condensing for applications. Short path evaporators are widely used in fine and specialty chemicals for thermal separation of intermediates, concentration of high value products, and molecular distillation under fine vacuum conditions. Their key features make them uniquely suitable for gentle evaporation and concentration of heat sensitive products at low pressures and temperatures.

It should be understood that many modifications of the above embodiments of the invention are possible within the scope of the invention such as the latter is defined in the appended claims.

### EXAMPLES

The invention will now be illustrated by means of the following nonlimiting examples. These examples are set forth merely for illustrative purposes and many other variations of the process may be used. The examples below summarizes some results from different purification of fish oils by molecular distillation.

### Equipment and conditions for laboratory experiments

In example 1-3 below decachlorobiphenyl, 0.60 mg/kg, was added to a fish oil composition as a pollutant model substance. The high chlorine content in decachlorobiphenyl ensures that this compound is less volatile than environmental pollutants like PCB, DDT and its metabolites, toxaphenes, dioxins and brominated flame retardants.

Unless otherwise stated, in all the examples the pressure was 0,001 mbar. However, as this is the lower limit of the pressure indicator, the real pressure will vary. That is the reason for somewhat varying results from one example to the next. When the distillation equipment is running under stable conditions, no significant variations are expected. However, this points out that constant pressure is not a very strong condition for carrying out the present invention.

### EXAMPLE 1: The effect of adding a working fluid

A fish oil composition containing fatty acids on triglyceride form and decachlorobiphenyl (0,60 mg/kg), with or without a working fluid, herein an ethyl ester, 8% relative to fish oil, (the ratio of (volatile working fluid):(fish oil) is about 8:100) was distilled by a laboratory scale molecular distillation at a rate of 600 ml/h and a temperature of 180°C. The used ethyl ester mixture was a by-product (distillate fraction) from production of EPA and DHA ethyl ester concentrates.

**Table 1: The effect of adding a volatile working fluid**

| | Decachlorobiphenyl (mg/kg) | Decachlorobiphenyl (% of start value) |
|---|---|---|
| Without WF | 0.43 | 72 |
| With WF | 0.022 | 3.7 |
| * WF = working fluid | | |

The results in table 1 show that addition of a volatile working fluid to a fish oil composition has a surprisingly and dramatic effect on the removal of decachlorobiphenyl. Here, more than 95% of the amount of decachlorobiphenyl has been removed ("stripped" off) from the fish oil mixture by molecular distillation.

### EXAMPLE 2: The effect of different flow rates.

A fish oil composition containing fatty acids in triglyceride form and decachlorobiphenyl (0,60 mg/kg) was added a working fluid in the form of a ethyl ester fraction in the same way as in example 1. The oil mixture was then stripped by a molecular distillation carried out at different flow rates, but at the same temperature (180 °C).

**Table 2: The effect of different flow rates**

| Flow rate (ml/h) | Decachlorobiphenyl (mg/kg) |
|---|---|
| 400 | 0.02 |
| 600 | 0.05 |
| 1000 | 0.20 |

The results given in the table above show that decachlorobiphenyl (and other volatile pollutants) will be flashed off (reduced) more successfully at lower flow rates. However, the results of optimising the flow rates are less important compared to the effect of adding a working fluid, such as a solvent, solvent mixture or a fraction containing an ethyl ester.

### EXAMPLE 3: The effect of different temperatures

Here, an ethyl ester fraction was added to a fish oil composition containing decachlorobiphenyl (0,60 mg/kg) in the same way as in example 1. The oil mixture was then stripped by molecular distillation at different temperatures.

**Table 3: The effect of different temperatures**

| Temperature (°C) | Decachlorobiphenyl (mg/kg) |
|---|---|
| 180 | 0.11 |
| 200 | 0.04 |

Table 3 illustrates that an increased temperature gives an improved removal of pollutants, when a volatile working fluid has been added to the oil mixture prior to a molecular distillation. Further, it is important to know that polyunsaturated fatty acids in fish oil are thermo-labile compounds and an increase in temperature is only applicable within strict limits.

For a person skilled in the art it is obvious that the same effect as described in example 1-3 will be achieved according to the invention by using other volatile working fluids, as long as the working fluids are essentially equally or less volatile than the environmental pollutants that is to be separated from the oil or fat mixture.

### EXAMPLE 4: Sardine oil - Industrial full scale process

This example shows an industrial scale process for decreasing the amount of pollutants in a fish oil mixture, which process comprises a step of adding a volatile working fluid to the fish oil mixture prior to a molecular distillation. 63.9 tons of a sardine oil containing different environmental pollutants was added a volatile working fluid in the form of a fatty acid ethyl ester mixture (ethyl ester of fish oil (8%)) before subjecting it to a molecular distillation process. The molecular distillation process was then carried out at a temperature of 200 °C, a pressure of 0,04 mbar and a mixture flow rate of 300 l/h with a heated surface of 3 m².

After treatment, 61.0 tons of purified product were collected. The results in table 4 show the content of vitamin A(trans-retinol), cholesterol, toxaphenes and dioxins in the sardine oil before and after stripping respectively.

**Table 4: Toxaphenes and dioxins in a sardine oil before and after stripping**

| | Before stripping | After stripping |
|---|---|---|
| Vitamin A | 15.3 mg/g | 13.0 mg/g |
| Cholesterol | 3.6 mg/g | 1.31 mg/g |
| Toxaphenes | 0.3 mg/g | <0.1 mg/g |
| Dioxins | 4.1 pg/g | 0.34 pg/g |

The results confirm that adding a working fluid to an oil before stripping is effective in reducing the amounts of volatile pollutants at the same time as the concentration of vitamin A, a valuable component in many fish oils, is not seriously affected. This means that this purification method can be used for products that contains vitamin A, e.g. cod liver oil.

In some cases a certain cholesterol level can be of value for some applications of fish oils e.g. for fish feed, especially feed for fish larvae. In these applications it is important to perform a preferential removal only of pollutants.

### EXAMPLE 5: Fish oil mixture - Industrial full scale process

This example also shows an industrial full scale process for decreasing the amount of pollutants in a fish oil, which process comprises the steps of adding a volatile working fluid to the fish oil mixture and subjecting the mixture, with the added volatile working fluid, to a molecular distillation processing step, in which environmental pollutants present in the fish oil is separated from the mixture with the volatile working fluid.

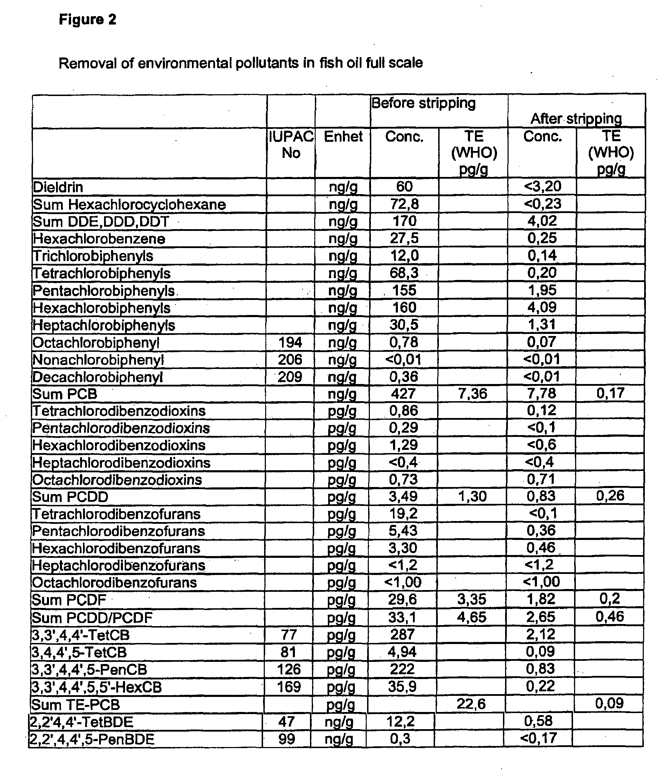

30 tons of a fish oil mixture containing different environmental pollutants (see Fig 2) was added a volatile working fluid in the form of an fatty acid ethyl ester mixture (ethyl ester of fish oil (6%)) before subjecting it to a molecular distillation process. The molecular distillation process was then carried out at a temperature of 200°C, a pressure of 0,005 mbar and a mixture flow rate of 400 kg oil/h with a heated surface of 11 m². After treatment, 29,5 tons of purified product were collected. The results are shown in Figure 2. The results confirm that the content of environmental pollutants in the fish oil mixture was strongly reduced after the stripping process according to the invention. For instance, the content of PCB in the fish oil mixture was reduced with about 98%, the content of PCDD was reduced with approximately 80%, the content of PCDF with about 95% and the amount of hexachlorocyclohexane respectively TE-PCB was almost negligible after stripping. For a person skilled in the art it is obvious that the same effect may be achieved according to the invention by using a volatile working fluid for decreasing an amount of pollutants in some other fat or oil compositions.

### EXAMPLE 6: Salmon oil

In this example oil from fresh by-products from Atlantic salmon was processed according to the invention. The process according to the invention comprises the steps of adding a volatile working fluid to the oil mixture and further subjecting the mixture, with the added volatile working fluid, to a molecular distillation processing step. 8% working fluid (the ratio of (volatile working fluid):(salmon oil) is here about 8:100) was added to the oil and the distillation process was performed at a pressure of 1x10⁻³ mbar, at a temperature of 180°C and at a mixture flow rate of 600 ml/hour.

Samples of the oil mixture was analysed before and after distillation respectively, regarding the amount of brominated flame retardants, PCBs and some chlorinated pesticides, see the tables 5 and 6 below.

**Table 5 (above): Brominated flame retardants, µg/kg, before and after a distillation process**

| Brominated flame retardants, µg/kg | Before treatment | After distillation |
|---|---|---|
| BDE 28 | 0.3 | < 0.2 |
| BDE 47 | 5.3 | < 0.2 |
| BDE 66 | 0.4 | < 0.2 |
| BDE 71 | < 0.2 | < 0.2 |
| BDE 75 | < 0.2 | < 0.2 |
| BDE 77 | < 0.2 | < 0.2 |
| BDE 85 | < 0.2 | < 0.2 |
| BDE 99 | 1.2 | < 0.2 |
| BDE 100 | 1.0 | < 0.2 |
| BDE 119 | < 0.2 | < 0.2 |
| BDE 138 | < 0.2 | < 0.2 |
| BDE 153 | < 0.2 | < 0.2 |
| BDE 154 | 0.5 | < 0.2 |
| BDE 183 | < 0.2 | < 0.2 |
| BDE190 | < 0.2 | < 0.2 |
| Me-TBBP-A | 0.2 | < 0.2 |
| HBCD | < 1.1 | < 1.2 |

**Table 6: PCB and chlorinated pesticides, µg/kg, before and after distillation**

| PCB and chlorinated pesticides, (µg/kg) | Before treatment | After distillation |
|---|---|---|
| CB 28 | < 3 | < 3 |
| CB 52 | 5 | < 3 |
| CB 101 | 11 | < 7 |
| CB 118 | < 9 | < 9 |
| CB 153 | 16 | < 7 |
| CB 105 | < 3 | < 3 |
| CB 138 | 13 | < 7 |
| CB 156 | < 3 | < 3 |
| CB 180 | 4.8 | < 4 |
| Dieldrin | 22 | < 4 |
| Endrin | < 3 | < 3 |
| HCB | 12 | < 1 |
| α-HCH | 3.8 | < 1 |
| γ-HCH | 5.3 | < 1 |
| β-HCH | < 5 | < 6 |
| β-HEPO | < 2 | < 3 |
| p,p'-DDE | 38 | < 3 |
| p,p'-DDD | 15 | < 3 |
| p,p'-DDT | nd = not detected | < 8 |

It is observed that the invention removes almost all of the analysed environmental pollutants to a level below the analytical detection limit.

Additionally, for a person skilled in the art it will be obvious that the same procedure could be used for other marine oils, for example cod liver oil or fish oil intended to be used as a component of feed for farmed fish. Nowadays, commercial fish feed contains high amounts of pollutants and it is therefore of large interest to decrease the amounts of toxic components in the current marine oil before the oil is added to the feed.

### EXAMPLE 7: Removal of PAH's

In this example benzo[a]pyrene (8.36 µg/kg oil) was added to a fish oil composition, that already had been treated according to the invention, in order to remove environmental pollutants. The fish oil composition containing the added benzo[a]pyrene was processed in a similar way and with the same distillation equipment as described in example 1. A volatile working fluid in the form of a fatty acid ethyl ester mixture, 8 % working fluid relative to the fish oil, was added to the fish oil composition, before subjecting the composition to a molecular distillation process. The molecular distillation process was carried out at a temperature of 180°C, a pressure of 1·10⁻³ mbar, and a mixture flow rate of 600 ml oil/h. The concentration of benzo[a]pyrene was analysed before processing and after processing, see table 7.

**Table 7: The amount of benzo[a]pyrene before and after processing**

| | Before processing | After processing |
|---|---|---|
| Benzo[a]pyrene (µg/kg) | 7,9 | 0,77 |

The results given in the table above show that benzo[a]pyrene will be flashed off successfully according to the invention. In this example the concentration of benzo[a]pyrene was reduced with about 90%.

This confirms that adding a volatile working fluid to a fat or an oil composition, at least containing polycyclic aromatic hydrocarbons (PAH's), before a stripping process according to the invention is effective in separating an amount of PAH's from the start fat or oil composition together with the volatile working fluid.

### EXAMPLE 8

A working fluid consisting of ethyl esters of fish oil (8%) was added to an oil produced from farmed salmon. A distillation process was carried out under the same conditions as in example 1 and a distillate fraction of 8.3% was collected. The acid value of the residual oil was reduced from 0.4 mgKOH/g before distillation to 0.1 mgKOH/g after distillation and the oil was analysed for contaminants before and after processing.

**Table 8: Salmon oil. Content of Indicator-PCBs (µg/kg) before and after 0 processning.**

| | CB-28 | CB-52 | CB-101 | CB-118 | CB-153 | CB-105 | CB-138 | CB-156 | CB-180 |
|---|---|---|---|---|---|---|---|---|---|
| Before | <3 | 5 | 11 | <9 | 16 | <3 | 13 | <3 | 4.8 |
| After | <3 | <3 | <7 | <9 | <7 | <3 | <7 | <3 | <4 |

**Table 9: Salmon oil. Content of organochlorine pesticides (µg/kg) before and after processing**

| | Dieldrin | Endrin | HCB | a-HCH | y-HCH | b-HCH | b-HEPO | pp_ DDE | pp_DDD | pp_DDT |
|---|---|---|---|---|---|---|---|---|---|---|
| Before | 22 | <3 | 12 | 3.8 | 5.3 | <6 | <3 | 38 | 15 | <8 |
| After | <4 | <3 | <1 | <1 | <1 | <6 | <3 | <3 | <3 | <8 |

The results show that adding a volatile working fluid prior to a stripping (distillation) process is effective in decreasing the amount of organo-chlorine pesticides in a fish oil composition. In addition, the volatile working fluid also facilitates removal of free fatty acids in the oil. Therein the acid value was decreased with 75%, i.e. from 0,4 to 0,1. It is hereby possibly to decrease the amount of environmental pollutants and to reduce the amount of free fatty acids in an oil or a fat at the same time and in the same process.

### EXAMPLE 9: Removal of free fatty acids

A fish oil purchased for production of fish feed was distilled by a molecular distillation process under the same conditions as given in example 1 and the start oil had an acid value of 6.8 mgKOH/g. After removal of a distillate corresponding to 4.3% by weight, the acid value of the residual oil was reduced to 0.2 mgKOH/g and the amount of environmental pollutants in the start oil was decreased.

In an identical distillation procedure, an oil with an acid value of 20.5 mgKOH/g was distilled. After removal of a distillate of 10.6 % the acid value was reduced to about 1.0 mgKOH/g and the amount of environmental pollutants in the start oil were decreased.

Due to the fact that the stripping process in example 8 also facilitates removal of free fatty acids in the oil and that the free fatty acids are volatile it can be expected that even oils with a low quality, i.e. a high content of free fatty acids, can be treated successfully according to the invention. An example of oils with low quality is silage oils or oils that have been stored or transported for a long period of time. Fish oils with low quality may be used for production of fish feed.

This example therefore shows that a stripping process for decreasing the amount of environmental pollutants in a mixture comprising at least a fat or an oil with a high content of free fatty acids (a low quality oil or fat) is effective since the free fatty acids in the oil or fat act as a working fluid. Further, the free fatty acids in the oil or fat also contributes to an additive effect in the stripping process by partially acting as an internal working fluid (or by being an active part of the working fluid) in the stripping process.

A person known in the art will also realise that the same stripping effect can be obtained by adding a volatile working fluid containing a similar volume of suitable free fatty acids to an oil or fat containing environmental pollutants in order to decrease the amount of environmental pollutants in the fat or oil.

### EXAMPLE 10: Removal of toxic components from a ricinus oil

This example shows a process for decreasing the amount of toxic compounds in a ricinus oil (castor oil, oil from the seeds of Ricinus communis L. Euphorbiaceae), which process comprises the steps of adding a volatile working fluid to a ricinus oil mixture and subjecting the mixture, with the added volatile working fluid, to a molecular distillation processing step, in which traces of ricinine from the ricinus oil is separated from the mixture with the volatile working fluid.

Firstly, 8% of a working fluid, (the ratio of (volatile working fluid):(ricinus oil) is here about 8:100) a distilled fraction of ethyl esters from a fish oil, was added to a ricinus oil prior to a distillation processing step. Secondly, the ricinus oil mixture was distilled in a molecular distillation process at a temperature of 170°C, at a pressure of 10⁻³ mbar and at a mixture flow of 500 ml/min. The concentration of toxic compounds were analysed before and after treatment. This analysis showed that the concentration of ricinine was substantially reduced compared to the concentration in the starting ricinus oil. This shows that it is possible to reduce traces of toxic components in a ricinus oil according to the invention, as long as the used volatile working fluid is essentially equally or less volatile than the components that is to be separated from the ricinus oil. Further, ricinus oil is used in both medicinal and cosmetic applications and the reduction of the existing trace levels of ricinin has commercial value.

The following items are part of the specification:
1. A process for decreasing the amount of environmental pollutants in a mixture comprising a fat or an oil, being edible or for use in cosmetics, the fat or oil containing the environmental pollutants, characterized in that the process comprises the steps of;
   - adding a volatile working fluid to the mixture, where the volatile working fluid comprises at least one of a fatty acid ester, a fatty acid amide, a free fatty acid and a hydrocarbon, and
   - subjecting the mixture with the added volatile working fluid to at least one stripping processing step, in which an amount of environmental pollutant present in the fat or oil, being edible or for use in cosmetics, is separated from the mixture together with the volatile working fluid.
2. A process according to item 1, wherein the volatile working fluid is essentially equally or less volatile than the environmental pollutants that are to be separated from the fat or oil mixture.
3. A process according to item 1, wherein the volatile working fluid is constituted by free fatty acids comprised in the fat or oil, being edible or for use in cosmetics, containing the environmental pollutants.
4. A process according to item 1, wherein the at least one of a fatty acid ester, a fatty acid amide and a free fatty acid is obtained from at least one of vegetable, microbial and animal fat or oil.
5. A process according to item 4, wherein the animal fat or oil is a fish oil and/or an oil obtained from sea mammals.
6. A process according to item 1, wherein the volatile working fluid comprises at least one fatty acid ester composed of C10-C22 fatty acids and C1-C4 alcohols, or a combination of two or more fatty acid ester each composed of C10-C22 fatty acids and C1-C4 alcohols.
7. A process according to item 1, wherein the fat or oil, being edible or for use in cosmetics, is obtained from at least one of vegetable, microbial and animal fat or oil, or any combination thereof.
8. A process according to item 7, wherein the fat or oil, being edible or for use in cosmetics, is a marine oil.
9. A process according to item 8, wherein the marine oil is obtained from fish or sea mammals, containing at least saturated and unsaturated fatty acids in the form of triglycerides.
10. A process according to item 7, wherein the fat or oil is a ricinus oil for use in cosmetics or medicinal applications.
11. A process according to item 7, wherein the fat or oil is a tocopherol concentrate prepared from a condensate from at least one deodorization process of at least one vegetable oil, wherein the tocopherol concentrate containing at least one of PAH and volatile pollutants, or any combination thereof.
12. A process according to item 1, wherein the ratio of (volatile working fluid):(fat or oil, being edible or for use in cosmetics) is about 1:100 to 15:100.
13. A process according to item 12, wherein the ratio of (volatile working fluid):(fat or oil, being edible or for use in cosmetics) is about 3:100 to 8:100.
14. A process according to item 1, wherein said stripping processing step is carried out at temperatures in the interval of 120-270 °C.
15. A process according to item 1, wherein said stripping processing step is carried out at temperatures in the interval of 150-200 °C.
16. A process according to item 1, wherein said stripping processing step is carried out at a pressure below 1 mbar.
17. A process according to item 1, wherein the at least one stripping processing step is one of a thin-film evaporation process, a molecular distillation or a short-path distillation or any combination thereof.
18. A process according to item 17, wherein the at least one thin-film evaporation process is carried out at a mixture flow rate in the interval of 10-300 kg/h·m².
19. A volatile environmental pollutants decreasing working fluid, for use in decreasing an amount of environmental pollutants present in a fat or oil, being edible or for use in cosmetics, the working fluid comprising at least one of a fatty acid ester, a fatty acid amide, a free fatty acid and a hydrocarbon, or any combination thereof.
20. A volatile environmental pollutants decreasing working fluid according to item 19, wherein said at least one of a fatty acid ester, a fatty acid amide, a free fatty acid is obtained from at least one of vegetable, microbial and animal origin, or any combination thereof.
21. A volatile fat or oil environmental pollutants decreasing working fluid according to item 20, wherein the animal origin is fish or sea mammals.
22. Use of a volatile environmental decreasing working fluid according to item 19, in a process for decreasing an amount of environmental pollutants, such as toxic component, in a mixture comprising a fat or oil, being edible or for use in cosmetics, preferably a marine oil, containing the toxic components, in which process the volatile working fluid is added to the mixture and then the mixture is subjected to at least one stripping processing step, preferably a thin-film evaporation process, a molecular distillation or a short-path distillation, or any combination thereof, and in which process an amount of toxic components present in the fat or oil, being edible or for use in cosmetics, is separated from the mixture together with the volatile working fluid.
23. A volatile environmental pollutants decreasing working fluid, wherein the volatile working fluid is a by-product, such as a distillate fraction, from a regular process for production of ethyl and/or methyl ester concentrates.
24. A health supplement, containing at least a marine oil, which marine oil is prepared according to the process presented in item1, in order to decrease the amount of environmental pollutants in the marine oil.
25. A pharmaceutical, containing at least a fish oil, which fish oil is prepared according to the process presented in item 1, in order to decrease the amount of environmental pollutants in the fish oil.
26. An animal feed product, containing at least a marine oil, which marine oil is prepared according to the process presented in item 1, in order to decrease the amount of environmental pollutants respectively the amount of free fatty acids in the marine oil.
27. An animal feed product according to item 26, wherein the feed product is a fish feed product.
28. A cosmetic product, based on ricinus oil, which ricinus oil is prepared according to the process presented in item 1, in order to decrease the amount of environmental pollutants in the ricinus oil.
29. A marine oil product, prepared according to the process presented in claim 1.
30. A marine oil product according to item 29, wherein the marine oil product is a fish oil product or a fish oil composition.
31. A tocopherol concentrate product, based on a tocopherol concentrate prepared from a condensate from a deodorization process of at least one vegetable fat or oil, such as palm oil or soy oil, which concentrate containing at least one of PAH and volatile pollutants and is prepared according to the process presented in item 1, in order to decrease the amount of PAH and volatile pollutants in the tocopherol concentrate.

## Claims

1. Use of a volatile environmental pollutants decreasing working fluid, for decreasing an amount of environmental pollutants present in a fat or oil, being edible or for use in cosmetics, in at least one stripping processing step, the working fluid consisting of a fatty acid ethyl ester, a fatty acid methyl ester or a fatty acid amide obtained from at least one of vegetable, microbial and animal origin, or any combination thereof, wherein the stripping processing step is a short-path distillation or a molecular distillation or any combination thereof and is carried out at temperatures in the interval of 150-270°C.

2. The use according to claim 1, wherein the fat or oil is a marine oil.

3. The use according to claim 2, wherein the marine oil is obtained from fish or sea mammals containing at least saturated and unsaturated fatty acids in the form of triglycerides.

4. The use of claim 1, wherein the environmental pollutants decreasing working fluid is volatile, wherein the volatile fluid particularly has a suitable volatility in relation to the volatility of the environmental pollutants, or wherein the volatile working fluid particularly is essentially equally or less volatile than the environmental pollutants.

5. The use according to claim 1, wherein the animal origin is fish or sea mammals.

6. The use according to claim 1, in a process for decreasing an amount of environmental pollutants, such as toxic component, in a mixture comprising a fat or oil, being edible or for use in cosmetics, preferably a marine oil, containing the toxic components, in which process the working fluid is added to the mixture and then the mixture is subjected to at least one stripping processing step, which is a molecular distillation or a short-path distillation, or any combination thereof, and in which process an amount of toxic components present in the fat or oil, being edible or for use in cosmetics, is separated from the mixture together with the working fluid.

7. The use of claim 1, wherein the working fluid is a by-product, such as a distillate fraction, from a regular process for production of ethyl and/or methyl ester concentrates.

8. The use of claim 1, wherein the oil or fat is for use as a health supplement, a pharmaceutical or an animal feed product.

9. The use of claim 1, wherein the environmental pollutants are selected from toxic components and/or pesticides like polychlorinated biphenyls (PCBs), DDT and its metabolites, polychlorinated triphehyls (PCTs), dibenzo-dioxins (PCDDs), and dibenzo-furans (PCDFs), chlorophenols and hexachlorocyclohexanes (HCHs), toxaphenes, dioxins, brominated flame retardants, polyaromatic hydrocarbons (PAH), organic tin-compounds and organic mercury-compounds.

## Patentansprüche

1. Verwendung eines flüchtigen Umweltschadstoffe verringernden Arbeitsfluids, zum Verringern der Menge von Umweltschadstoffen, die in einem Fett oder Öl vorhanden sind, das verzehrbar oder zur Verwendung in Kosmetika ist, in mindestens einem Abstreif-Verfahrensschritt, wobei das Arbeitsfluid aus einem Fettsäureethylester, einem Fettsäuremethylester oder einem Fettsäureamid besteht, der/das aus mindestens einem pflanzlichen, mikrobiellen und tierischen Ursprung erhalten ist, oder einer beliebigen Kombination davon, wobei der Abstreif-Verfahrensschritt eine Kurzwegdestillation oder eine molekulare Destillation oder beliebige Kombination davon ist, und bei Temperaturen im Bereich von 150-270°C durchgeführt wird.

2. Verwendung nach Anspruch 1, wobei das Fett oder Öl ein marines Öl ist.

3. Verwendung nach Anspruch 2, wobei das marine Öl aus Fisch oder Meeressäugern erhalten ist und wenigstens gesättigte und ungesättigte Fettsäuren in der Form von Triglyceriden enthält.

4. Verwendung von Anspruch 1, wobei das Umweltschadstoffe verringernde Arbeitsfluid flüchtig ist, wobei das flüchtige Fluid insbesondere eine geeignete Flüchtigkeit in Beziehung zu der Flüchtigkeit der Umweltschadstoffe aufweist, oder wobei das flüchtige Arbeitsfluid insbesondere im Wesentlichen gleich oder weniger flüchtig ist als die Umweltschadstoffe.

5. Verwendung nach Anspruch 1, wobei der tierische Ursprung Fisch oder Meeressäuger ist.

6. Verwendung nach Anspruch 1, in einem Verfahren zum Verringern der Menge von Umweltschadstoffen, wie z.B. toxischer Komponente, in einem Gemisch, das ein Fett oder Öl umfasst, das verzehrbar oder zur Verwendung in Kosmetika ist, vorzugsweise ein marines Öl, das die toxischen Komponenten enthält, wobei bei dem Verfahren das Arbeitsfluid zu dem Gemisch zugegeben wird und anschließend das Gemisch wenigstens einem Abstreif-Verfahrensschritt unterzogen wird, das eine molekulare Destillation oder eine Kurzwegdestillation, oder beliebige Kombination davon ist, und wobei bei dem Verfahren eine Menge an toxischen Komponenten, die in einem Fett oder Öl enthalten sind, das verzehrbar oder zur Verwendung in Kosmetika ist, zusammen mit dem flüchtigen Arbeitsfluid von dem Gemisch abgetrennt wird.

7. Verwendung von Anspruch 1, wobei das Arbeitsfluid ein Nebenprodukt, wie z.B. eine Destillatfraktion, eines gängigen Verfahrens zur Herstellung von Ethyl- und/oder Methylester-Konzentraten ist.

8. Verwendung von Anspruch 1, wobei das Öl oder Fett zur Verwendung als eine Gesundheitsergänzung, ein pharmazeutisches oder ein Tierfutter-Produkt ist.

9. Verwendung nach Anspruch 1, wobei die Umweltschadstoffe ausgewählt sind aus toxischen Komponenten und/oder Pestiziden wie polychlorierte Biphenyle (PCBs), DDT und seinen Metaboliten, polychlorierte Triphenyle (PCTs), Dibenzodioxine (PCDDs), und Dibenzofurane (PCDFs), Chlorphenole und Hexachlorocyclohexane (HCHs), Toxaphene, Dioxine, bromierte Flammschutzmittel, polyaromatische Kohlenwasserstoffe (PAH), organische Zinnverbindungen und organische Quecksilberverbindungen.

## Revendications

1. Utilisation d'un fluide actif volatil capable de diminuer les polluants environnementaux, pour diminuer une quantité de polluants environnementaux présents dans une matière grasse ou une huile, qui est comestible ou à utiliser en cosmétique, dans au moins une étape opératoire d'extraction, le fluide actif consistant en un ester éthylique d'acide gras, un ester méthylique d'acide gras ou un amide d'acide gras obtenu à partir d'au moins une origine végétale, microbienne et animale, ou une quelconque combinaison de ceux-ci, où l'étape opératoire d'extraction est une distillation à trajet court ou une distillation moléculaire ou une quelconque combinaison de celles-ci et est effectuée à des températures dans l'intervalle de 150-270°C.

2. Utilisation selon la revendication 1, dans laquelle la matière grasse ou l'huile est une huile marine.

3. Utilisation selon la revendication 2, dans laquelle l'huile marine est obtenue à partir de poissons ou de mammifères marins contenant au moins des acides gras saturés et insaturés sous la forme de triglycérides.

4. Utilisation selon la revendication 1, dans laquelle le fluide actif capable de diminuer les polluants environnementaux est volatil, dans laquelle le fluide volatil a en particulier une volatilité convenable par rapport à la volatilité des polluants environnementaux, ou dans laquelle le fluide actif volatil est en particulier essentiellement aussi volatil ou moins volatil que les polluants environnementaux.

5. Utilisation selon la revendication 1, dans laquelle l'origine animale consiste en poissons ou mammifères marins.

6. Utilisation selon la revendication 1, dans un procédé pour diminuer une quantité de polluants environnementaux, comme un composant toxique, dans un mélange comprenant une matière grasse ou une huile, qui est comestible ou à utiliser en cosmétique, de préférence une huile marine, contenant les composants toxiques, procédé dans lequel le fluide actif est ajouté au mélange et ensuite le mélange est soumis à au moins une étape opératoire d'extraction, qui est une distillation moléculaire ou une distillation à trajet court, ou une quelconque combinaison de celles-ci, et procédé dans lequel une quantité de composants toxiques présents dans la matière grasse ou l'huile, qui est comestible ou à utiliser en cosmétique, est séparée du mélange en même temps que le fluide actif.

7. Utilisation selon la revendication 1, dans laquelle le fluide actif est un sous-produit, tel qu'une fraction de distillat, d'un procédé courant de production de concentrés d'esters éthyliques et/ou méthyliques.

8. Utilisation selon la revendication 1, dans laquelle l'huile ou la matière grasse est destinée à l'utilisation comme supplément de santé, comme produit pharmaceutique ou comme produit alimentaire pour animaux.

9. Utilisation selon la revendication 1, dans laquelle les polluants environnementaux sont choisis parmi les composants toxiques et/ou les pesticides comme les biphényles polychlorés (PCB), le DTT et ses métabolites, les triphényles polychlorés (PCT), les dibenzodioxines (PCDD) et les dibenzofuranes (PCDF), les chlorophénols et les hexachlorocyclohexanes (HCH), les toxaphènes, les dioxines, les retardateurs de flamme bromés, les hydrocarbures polyaromatiques (PAH), les composés organo-stanniques et les composés de mercure organiques.
